**Europäisches Patentamt**

**European Patent Office**

**Office européen des brevets**

(19)

(11) Veröffentlichungsnummer : **0 087 711**
**B1**

(12) **EUROPÄISCHE PATENTSCHRIFT**

(45) Veröffentlichungstag der Patentschrift :
16.04.86

(51) Int. Cl.⁴ : **C 07 C143/12**, C 07 C139/12,
B 01 F 17/10

(21) Anmeldenummer : 83101638.1

(22) Anmeldetag : 21.02.83

(54) Verfahren zur Herstellung von Sulfobernsteinsäure-Diestern.

(30) Priorität : 01.03.82 DE 3207222

(43) Veröffentlichungstag der Anmeldung :
07.09.83 Patentblatt 83/36

(45) Bekanntmachung des Hinweises auf die Patenterteilung : 16.04.86 Patentblatt 86/16

(84) Benannte Vertragsstaaten :
AT BE CH DE FR GB IT LI NL SE

(56) Entgegenhaltungen :
BE-A- 487 957
DE-A- 1 468 023
DE-A- 1 912 531
DE-A- 2 127 608

(73) Patentinhaber : Henkel Kommanditgesellschaft auf
Aktien
Postfach 1100 Henkelstrasse 67
D-4000 Düsseldorf-Holthausen (DE)

(72) Erfinder : Höfer, Rainer, Dr.
Klever Strasse 31
D-4000 Düsseldorf 30 (FR)
Erfinder : Ackermann, Karl-Heinz
Elisabethstrasse 117
D-4000 Düsseldorf 1 (DE)

## Beschreibung

Die Erfindung betrifft ein verbessertes Verfahren zur Herstellung von Sulfobernsteinsäure-Dialkylestern durch Umsetzung von Maleinsäuredialkylestern mit 4 bis 18 Kohlenstoffatomen pro Alkylrest in wässrig-organischem Medium bei Temperaturen unter 100 °C mit Alkalidisulfiten oder Alkalihydrogensulfiten.

Die älteren Methoden zur Herstellung von Sulfobernsteinsäuredialkylestern sind in K. Lindner « Tenside — Textilhilfsmittel — Waschrohstoffe », Bd. I, S. 747 ff, Stuttgart 1964, beschrieben. Sie beruhen darauf, daß eine wässrig-alkalische Hydrogensulfit-Lösung und Maleinsäurediester unter Rühren und Rückfluß auf ca 70 bis 100 °C erwärmt werden. Es ist bekannt, daß bei dieser Arbeitsweise beachtliche Mengen $SO_2$ aus der $NaHSO_3$-Lösung abgespalten werden. Sie gehen damit — falls man nicht in einem geschlossenen System unter Überdruck arbeitet — für die Reaktion verloren und führen wegen des intensiven stechenden Geruchs zu einer erheblichen Arbeitsplatzbelastung.

Die DE-AS-1 213 686 beschreibt Haut- und Haarpflegemittel auf Basis von Wollfett mit einem Gehalt an Sulfobernsteinsäureestern, wobei das Herstellungsverfahren der Bernsteinsäureester der vorstehend beschriebenen Verfahrensweise entspricht.

Die DE-AS-1 912 531 beschreibt ein Verfahren zur Herstellung von Sulfobernsteinsäuredialkylester-Salzen, wobei der Zusatz des sauren Sulfits durch schrittweises Einleiten einer wässrigen Lösung dieses Sulfits erfolgt und in homogenem Milieu in Gegenwart eines Homogenisierungsmittels gearbeitet wird. Es werden dort Reaktionszeiten von mehr als 9 Stunden benötigt.

Die DE-OS-2 546 847 beschreibt ein Verfahren zur Herstellung von Alkylsulfosuccinaten, bei dem für die Sulfitierungsreaktion ein bestimmter pH-Wert eingestellt und unter Mitverwendung von Emulgatoren zwecks Homogenisierung des Reaktionsgemisches gearbeitet wird.

Ebenfalls unter Kontrolle des pH-Wertes bei der Sulfitierung arbeitet das in der DE-OS-2 606 611 beschriebene Verfahren. Dabei wird eine wässrige, Maleinsäurediester und Natriumdisulfitlösung enthaltende Mischung auf einen pH von 4,5 bis 6 eingestellt. Im Verlauf der Reaktion steigt der pH auf 6,5 bis 8,5. Dieser pH-Anstieg in den basischen pH-Bereich muss durch Zusatz von $H^+$-Ionen gestoppt werden. Dies geschieht z. B. durch Zugabe starker oder schwacher Säuren. Damit wird jedoch der Elektrolytgehalt der wässrigen Sulfosuccinatlösung erhöht. In einer Reihe von Anwendungen, insbesondere als Emulgator in der Emulsionspolymerisation, sind jedoch besonders salzarme Formulierungen gewünscht. Außerdem erfordert das in der DE-OS-2 606 611 beschriebene Verfahren eine sehr genaue Überwachung der Reaktion. Wird nämlich die Umsetzung bei Erreichen eines pH von 7,5 bis 8,5 nicht sofort abgestoppt, so erfolgt in sehr kurzer Zeit ein weiterer pH-Anstieg in den stark basischen Bereich und dadurch bedingt eine basenkatalysierte Spaltung der Estergruppierung. Das in der DE-OS-2 606 611 beschriebene Verfahren hat also den Nachteil, daß es Formulierungen mit u. U. unerwünscht hohen Elektrolytanteilen schafft und in seinem Ablauf sehr genau überwacht werden muß, also personalintensiv ist.

Die BE-A-487 957 beschreibt ein Verfahren zur Herstellung von organischen Sulfonaten durch Addition eines Bisulfitions an eine organische Verbindung, die eine oder mehrere olefinische Doppelbindungen enthält. Die Reaktion wird in Gegenwart eines Reaktionsinitiators und gegebenenfalls eines Lösungsmittels bei Temperaturen zwischen 50 und 200 °C durchgeführt. Als Initiatoren für die Reaktion kommen insbesondere in Betracht: 2,2-Bis-(tert.-butylperoxy)-butan in Kombination mit Methanol oder Pyridin, tert.-Butylperbenzoat in Kombination mit Methanol, Ethanol, Hexanolamin oder Pyridin sowie Di-tert.-butylperoxid mit Pyridin. Als geeignete Substanzen für die Bisulfitanlagerung werden ausschließlich olefinisch ungesättigte Kohlenwasserstoffe mit 4 bis 20 Kohlenstoffatomen genannt, wobei 1-Alkene von ganz besonderem Interesse sind. Organische Verbindungen, die neben olefinischen Doppelbindungen auch noch funktionelle Gruppen enthalten, insbesondere solche, die Estergruppierungen in unmittelbarer Nachbarschaft zur Doppelbindung enthalten, werden nicht erwähnt.

Der vorliegenden Erfindung lag die Aufgabe zugrunde, ein Verfahren für die Herstellung von Sulfobernsteinsäure-Diestern mit 4 bis 18 Kohlenstoffatomen im Alkylrest zu finden, das in kurzer Zeit ohne besondere Überwachungsmaßnahmen während der Reaktion abläuft und ohne Verwendung von Emulgatoren oder anderen Homogenisierungsmitteln salzarme und damit für die Emulsionspolymerisation besonders geeignete Produkte liefert.

Es wurde überraschend gefunden, daß die gestellte Aufgabe sehr einfach gelöst werden kann, wenn die Sulfitierungsreaktion in Gegenwart eines Katalysators durchgeführt wird.

Gegenstand der vorliegenden Erfindung ist demgemäß ein Verfahren zur Herstellung von Sulfobernsteinsäure-Diestern durch Umsetzung von Maleinsäuredialkylestern mit 4 bis 18 Kohlenstoffatomen pro Alkylrest in wässrigorganischem Medium bei Temperaturen unter 100 °C mit Alkalidisulfiten oder Alkalihydrogensulfiten, das dadurch gekennzeichnet ist, daß die Sulfitierungsreaktion in Gegenwart eines zugefügten, unter diesen Bedingungen freie Radikale bildenden Katalysators ausgewählt aus der Gruppe bestehend aus Azoverbindungen, Azobisverbindungen, Peroxiverbindungen und Verbindungen, bei denen die freien Radikale durch Spaltung an Kohlenstoff-Kohlenstoff-Bindungen gebildet werden, durchführt.

Als Katalysatoren, die unter den Verfahrensbedingungen freie Radikale bilden, kommen Azo- und/oder Azobis-Verbindungen infrage, wie sie üblicherweise bei der radikalischen Polymerisation als Initiatoren eingesetzt werden. Als Beispiele seien 2,2'-Azo-bis-2-methylpropionitril (Azo-bis-isobutyronitril), 2,2'-Azo-bis-2-methylbutyronitril, 2,2'-Azo-bis-2,3-dimethylbutyronitril, 2,2'-Azo-bis-2-methylvaleronitril, 2,2'-Azo-bis-2,4-dimethylvaleronitril, 1,1'-Azo-bis-1-cyanocyclohexan, 2,2'-Azo-bis-2-benzylpropionitril, 4,4'-Azo-bis-4-cyano-1-methylpiperidin, 2,2'-Azo-bis-(methylisobutyrat), 2,2'-Azo-bis-(ethylisobutyrat), 1,1'-Azo-bis-cumen, 1,1'-Azo-bis-1-phenyl-ethan und Phenyl-azo-triphenylmethan. Als besonders geeignet haben sich Azo-bis-isobutyronitril und 2,2'-Azo-bis-(ethylisobutyrat) erwiesen.

Als Katalysatoren, die unter den Reaktionsbedingungen freie Radikale bilden, kommen weiterhin die Peroxide infrage, die üblicherweise bei Reaktionen verwendet werden, bei denen freie Radikale gebildet werden. Als Beispiele seien Diacetylperoxid, Dipropionylperoxid, Diisobutyrylperoxid, Dipelargonylperoxid, Dilauroylperoxid, Dibenzoylperoxid, Di-p-chlorbenzoylperoxid, Di-2, 3-dichlorbenzoylperoxid, Methyläthylketonperoxid, Di-tert.-butylperoxid, Dicumylperoxid, tert.-Butylperoxypivalat, tert.-Butylperoxyoctoat, tert.-Butylperoxybenzoat und tert.-Butylperoxyphthalat genannt.

Ferner können als Katalysatoren solche Verbindungen verwendet werden, bei denen die Radikale durch Spaltung an Kohlenstoff-Kohlenstoffbindungen gebildet werden. Als Beispiele seien 2, 3-Dimethyl-2, 3-diphenylbutan, genannt.

Der Katalysator wird vorzugsweise in einer Menge von 0, 01 bis 10 Gewichtsprozent, insbesondere von 0, 05 bis 5 Gewichtsprozent, bezogen auf eingesetzten Maleinsäurediester, verwendet.

Die Alkylreste der eingesetzten Maleinsäuredialkylester können gleich oder verschieden und geradekettig oder verzweigt oder cyclisch sein. Die Länge oder Art des Alkylrestes spielt für die Durchführung des Verfahrens der vorliegenden Erfindung keine Rolle. Welche Alkylreste im Ausgangsmaterial gewählt werden, hängt weitgehend von der späteren Verwendung der Sulfobernsteinsäure-Dialkylester ab, was dem Fachmann bekannt ist, beispielsweise aus dem Werk von K. Lindner « Tenside — Textilhilfsmittel — Wachrohstoffe » loc. cit.

Die für die Sulfitierungsreaktion angewandten Temperaturen liegen bei 60 bis unter 100 °C, vorzugsweise bei 70 bis 95 °C und insbesondere bei 80 bis 85 °C.

Die Reaktionszeit beträgt im allgemeinen 2 bis 4 und insbesondere 2, 5 bis 3, 5 Stunden, liegt jedoch in einigen Fällen auch bei 4 bis 6 Stunden. Dies hängt naturgemäss von den eingesetzten Materialien ab.

Das erfindungsgemässe Verfahren ist äußerst einfach. Alle Reaktanten, Wasser, gegebenenfalls ein organisches Lösungsmittel und die gesamte Katalysatormenge werden zu Beginn der Reaktion auf einmal gemischt und dann beispielsweise während 2, 5 bis 3, 5 Stunden auf 80 bis 85 °C erhitzt.

Als organische Lösungsmittel kommen solche infrage, die die Sulfitierungsreaktion nicht negativ beeinflussen. Beispielsweise sind dies Alkanole wie Ethanol, Propanol und Butanol.

In Gegenwart von gewissen Mengen organischer Lösungsmittel ist das Ende der Sulfitierungsreaktion sehr einfach am Klarwerden des vorher trüben Ansatzes zu erkennen.

Die Vorteile des erfindungsgemässen Verfahrens liegen darin, daß es sehr einfach durchzuführen ist, daß eine Einstellung oder Kontrolle des pH-Wertes und auch eine Verwendung von Emulgatoren nicht erforderlich ist. Es wird jedoch nicht ausgeschlossen, daß die Mitverwendung von vorher gefertigtem Endprodukt (d. h. von Sulfobernsteinsäurediester) als Homogenisierungsmittel in bestimmten Fällen vorteilhaft sein kann.

Die nach dem erfindungsgemässen Verfahren hergestellten Sulfobernsteinsäure-Diester haben dem Fachmann bekannte Anwendungsgebiete und können z. B. als Emulgatoren oder als Netzmittel verwendet werden.

Die Erfindung wird nachstehend anhand von Ausführungsbeispielen erläutert :

Beispiel 1

In einem 2-l-Dreihalskolben mit Rührer, Rückflußkühler und Thermometer wurde eine Lösung von 143 g Natriumdisulfit in 321,5 g Wasser vorgelegt, 408 g Di-n-hexylmaleinat, 124 g Ethanol und 3,5 g Azo-bis-isobutyronitril (ABIN) wurden hinzugegeben. Unter intensivem Rühren wurde auf 80 bis 85 °C aufgeheizt und 2,5 bis 3,5 Stunden weitergerührt. Nach dieser Zeit lag eine klare Lösung vor und die Reaktion war beendet.

Nach einer halbstündigen Nachrührphase wurde der Ansatz auf Raumtemperatur abgekühlt. Das während der Reaktion zusammengeklumpte AIBN wurde über einen groben Filter abgetrennt. Man erhielt eine wasserhelle bis schwach gelbliche, klare Lösung von Di-n-hexylsulfosuccinat in Wasser/Ethanol. Die Ausbeute, bezogen auf eingesetzten Ester, lag bei 98 bis 100 %.

Beispiel 2

In der in Beispiel 1 beschriebenen Apparatur wurde eine Lösung von 157,7 g Natriumdisulfit in 200 g Wasser vorgelegt, 552,3 g Di-2-Ethylhexylmaleinat, 86,5 g Ethanol und 3,5 g AIBN wurden hinzugegeben. Unter intensivem Rühren wurde auf 80 bis 85 °C aufgeheizt und 2,5 bis 3,5 Stunden gerührt. Auch hier wurde das Ende der Reaktion am Klarwerden des Ansatzes erkennbar. Die weitere Behandlung erfolgte wie in Beispiel 1 beschrieben. Man erhielt eine klare Lösung von Di-2-Ethylhexylsulfosuccinat in Wasser/Ethanol.

Die Ausbeute, bezogen auf eingesetzten Ester, lag bei 98 bis 100 %.

Beispiel 3

Der Versuch von Beispiel 1 wurde mit folgenden Mengen folgender Produkte wiederholt: 179 g Natriumdisulfit in 224 g Wasser, 510 g Di-iso-hexylmaleinat, 83 g Ethanol und 4 g AIBN. Nach dem in Beispiel 1 beschriebenen Verfahren erhielt man eine leicht gelbliche, klare Lösung von Di-iso-hexylsulfosuccinat in Wasser/Ethanol. Die Ausbeute, bezogen auf eingesetzten Ester, lag bei 96 bis 100 %.

Beispiel 4

Der Versuch von Beispiel 2 wurde mit folgenden Mengen folgender Produkte wiederholt: 160 g Natriumdisulfit in 190 g Wasser, 561 g Di-2-Ethylhexylmaleinat, 85 g Ethanol und 4 g 2,3-Dimethyl-2,3-diphenylbutan.
Nach dem in Beispiel 1 beschriebenen Verfahren erhielt man eine leicht gelbliche, klare Lösung von Di-2-Ethylhexylsulfosuccinat in Wasser/Ethanol.

Beispiel 5

Der Versuch von Beispiel 2 wurde mit folgenden Mengen folgender Produkte wiederholt: 160 g Natriumdisulfit in 190 g Wasser, 561 g Di-2-Ethylhexylmaleinat, 85 g Ethanol und 4 g 2,2'-Azo-bis-(ethylisobutyrat). Auch bei diesem Versuch wurde unter intensivem Rühren auf 80 bis 85 °C aufgeheizt und 2,5 bis 3,5 Stunden gerührt. Das Ende der Reaktion wurde am Klarwerden des Ansatzes erkennbar. Bei diesem Versuch fiel Di-2-Ethylhexylsulfosuccinat rückstansfrei in Form einer gelbliche-klaren Lösung in Wasser/-Ethanol an.

Beispiel 6

Der Versuch von Beispiel 2 wurde mit folgenden Mengen folgender Produkte wiederholt: 160 g Natriumdisulfit in 190 g Wasser, 561 g Di-2-Ethylhexylmaleinat, 85 g Ethanol und 4 g Dilauroylperoxid.
Bei diesem Verfahren betrug die Reaktionsdauer bis zum Klarwerden des Ansatzes 5,5 bis 6 Stunden und lag damit ohne Verwendung von Homogenisierungsmitteln unter den in DE-AS-1 912 531 genannten Reaktionszeiten.

Beispiel 7

Der Versuch von Beispiel 2 wurde mit folgenden Mengen folgender Produkte wiederholt: 160 g Natriumdisulfit in 190 g Wasser, 561 g Di-2-Ethylhexylmaleinat, 4 g AIBN und 85 g n-Propanol. Diesmal wurde das Reaktionsgemisch ebenfalls unter intensivem Rühren auf 90 bis 95 °C aufgeheizt. Nach 2,5 bis 3 Stunden war das Ende der Reaktion am Klarwerden des Ansatzes zu erkennen. Die weitere Behandlung erfolgte wie in Beispiel 1 beschrieben. Man erhielt eine klare Lösung von Di-2-Ethylhexylsulfosuccinat in Wasser/n-Butanol. Die Ausbeute, bezogen auf eingesetzten Ester, lag bei 98 bis 100 %.

**Patentansprüche**

1. Verfahren zur Herstellung von Sulfobernsteinsäure-Diestern durch Umsetzung von Maleinsäuredialkylestern mit 4 bis 18 Kohlenstoffatomen pro Alkylrest in wäßrig-organischem Medium bei Temperaturen unter 100 °C mit Alkalidisulfiten oder Alkalihydrogensulfiten, dadurch gekennzeichnet, daß man die Sulfitierungsreaktion in Gegenwart eines zugefügten, unter diesen Bedingungen freie Radikale bildenden Katalysators ausgewählt aus der Gruppe bestehend aus Azoverbindungen, Azobisverbindungen, Peroxiverbindungen und Verbindungen, bei denen die freien Radikale durch Spaltung an Kohlenstoff-Kohlenstoff-Bindungen gebildet werden, durchführt.

2. Verfahren nach Anspruch 1, dadurch gekennzeichnet, daß man als Katalysatoren Azobis-Isobutyronitril verwendet.

3. Verfahren nach Anspruch 1, dadurch gekennzeichnet, daß man als Katalysator 2,2' -Azo-bis(ethylisobutyrat) verwendet.

4. Verfahren nach den Ansprüchen 1 bis 3, dadurch gekennzeichnet, daß man die Katalysatoren in einer Menge von 0,01 bis 10 Gew.-%, vorzugsweise 0,05 bis 5 Gew.-%, bezogen auf eingesetzten Maleinsäure-Diester, verwendet.

5. Verfahren nach den Ansprüchen 1 bis 4, dadurch gekennzeichnet, daß die Alkylreste der Maleinsäuredialkylester gleich oder verschieden und geradkettig oder verzweigt oder cyclisch sein können.

6. Verfahren nach den Ansprüchen 1 bis 5, dadurch gekennzeichnet, daß ein organisches Lösungsmittel mitverwendet wird.

7. Verwendung der nach Ansprüchen 1 bis 6 erhaltenen Sulfobernsteinsäure-Diester als Emulgatoren oder als Netzmittel.

**Claims**

1. A process for the production of sulfosuccinic acid diesters by reaction of maleic acid dialkylesters containing from 4 to 18 carbon atoms per alkyl group with alkali disulfites or alkali hydrogen sulfites in aqueous-organic medium at temperatures below 100 °C, characterized in that the sulfiting reaction is carried out in the presence of an added catalyst forming free radicals under these conditions selected from the group comprising azo compounds, azo-bis compounds, peroxy compounds and compounds in which the free radicals are formed by cleavage at carbon-carbon bonds.

2. A process as claimed in Claim 1, characterized in that azo-bis-isobutyronitrile is used as catalyst.

3. A process as claimed in Claim 1, characterized in that 2,2'-azo-bis-(ethylisobutyrate) is used as catalyst.

4. A process as claimed in Claims 1 to 3, characterized in that the catalysts are used in a quantity of from 0.01 to 10 % by weight and preferably in a quantity of from 0.05 to 5 % by weight, based on the maleic acid diester used.

5. A process as claimed in Claims 1 to 4, characterized in that the alkyl groups of the maleic acid diester may be the same or different and may be linear or branched or cyclic.

6. A process as claimed in Claims 1 to 5, characterized in that an organic solvent is used.

7. The use of the sulfosuccinic acid diesters obtained by the process claimed in Claims 1 to 6 as emulsifiers or wetting agents.

**Revendications**

1. Procédé de préparation de diesters de l'acide sulfosuccinique par réaction d'esters dialkyliques de l'acide maléique contenant 4 à 18 atomes de carbone par radical alkyle, dans un milieu organique aqueux et à des températures inférieures à 100 °C, avec des disulfites alcalins ou des hydrogénosulfites alcalins, caractérisé en ce que la réaction de sulfitation est effectuée en présence d'un catalyseur ajouté formant des radicaux libres dans ces conditions et choisi parmi le groupe comprenant les azo-composés, les azo-bis-composés, les peroxy-composés et les composés dans lesquels les radicaux libres sont formés par dissociation sur des liaisons carbone-carbone.

2. Procédé selon la revendication 1, caractérisé en ce que, comme catalyseur, on utilise l'azo-bis-isobutyronitrile.

3. Procédé selon la revendication 1, caractérisé en ce que, comme catalyseur, on utilise le 2,2'-azo-bis-(éthylisobutyrate).

4. Procédé selon les revendications 1 à 3, caractérisé en ce qu'on utilise les catalyseurs en une quantité de 0,01 à 10 % en poids, de préférence, de 0,05 à 5 % en poids, calculé sur le diester d'acide maléique utilisé.

5. Procédé selon les revendications 1 à 4, caractérisé en ce que les radicaux alkyle des esters dialkyliques de l'acide maléique peuvent être identiques ou différents, à chaîne droite, à chaîne ramifiée ou cycliques.

6. Procédé selon les revendications 1 à 5, caractérisé en ce qu'on utilise conjointement un solvant organique.

7. Utilisation des diesters de l'acide sulfosuccinique obtenus selon les revendications 1 à 6 comme émulsionnants ou comme agents mouillants.